# EUROPÄISCHE PATENTSCHRIFT

(11) **EP 3 294 216 B1**
(45) Veröffentlichungstag und Bekanntmachung des Hinweises auf die Patenterteilung: **05.07.2023**
(21) Anmeldenummer: 16726276.5
(22) Anmeldetag: 10.05.2016
(51) Int. Cl.: A61F 2/24

(54) **IMPLANTIERBARE VORRICHTUNG ZUR VERBESSERUNG ODER BEHEBUNG EINER HERZKLAPPENINSUFFIZIENZ**
IMPLANTABLE DEVICE FOR IMPROVING OR REMEDYING VALVULAR INCOMPETENCE
DISPOSITIF IMPLANTABLE POUR AMÉLIORER OU SUPPRIMER UNE INSUFFISANCE VALVULAIRE

(30) Priorität: 12.05.2015 DE 102015005933
(43) Veröffentlichungstag der Anmeldung: 21.03.2018
(73) Patentinhaber: Universität Duisburg-Essen, 45141 Essen (DE); coramaze technologies GmbH, 40721 Hilden (DE)
(72) Erfinder: NEUMANN, Till, 44869 Bochum (DE); SCHEUERMANN, Torsten, 80803 München (DE); NATHE, Niklas, 40227 Düsseldorf (DE)
(74) Vertreter: Dennemeyer & Associates S.A.
(86) Internationale Anmeldenummer: PCT/EP2016/000757
(87) Internationale Veröffentlichungsnummer: WO 2016/180529

(56) Entgegenhaltungen:
- EP-A1- 2 762 190
- EP-B1- 2 854 710
- US-A- 6 053 922
- US-A1- 2005 177 131
- US-A1- 2007 293 943
- US-A1- 2009 048 668
- US-A1- 2011 034 764
- US-A1- 2013 325 110
- US-A1- 2015 073 547

## Beschreibung

Die Erfindung betrifft eine implantierbare Vorrichtung zur Verbesserung oder Behebung einer Herzklappeninsuffizienz umfassend einen Verschlusskörper und wenigstens ein daran befestigtes Verankerungselement, mit dem der Verschlusskörper im Herzen, bevorzugt im Atrium des Herzens befestigbar ist.

Implantierbare Vorrichtungen dieser genannten Art sind im Stand der Technik bekannt und werden eingesetzt wenn bei einem Patienten während der üblichen Herztätigkeit die Herzklappen nicht genügend schließen und hierdurch Fehlströmungen zwischen Ventrikel und Atrium des Herzens entstehen.

Eine implantierbare Vorrichtung der eingangs genannten Art wird sodann so im Herzen des Patienten implantiert, so dass der Verschlusskörper im Durchgangsbereich der Herzklappe angeordnet ist und sich somit die Segelklappen der Herzklappe beim Schließen an den Verschlusskörper außen anlegen, demnach also der eingesetzte Verschlusskörper einen ansonsten verbleibenden Spaltbereich verschließt. Durch den Verschlusskörper einer solchen implantierbaren Vorrichtung wird somit ein verbessertes Schließen der Herzklappe wieder hergestellt und die zuvor genannten Fehlströmungen verhindert.

Die Befestigung einer solchen Vorrichtung mittels des daran befestigten Verankerungselementes kann dabei so erfolgen, dass das Verankerungselement am Verschlusskörper eine atraumatische Befestigung im Herzen ermöglicht, insbesondere dadurch, dass sich das Verankerungselement zum Zweck der Befestigung an die Innenwand des Herzens anlegt, ohne die Herzwandung zu penetrieren.

Beispielsweise kann ein Verankerungselement hierfür durch ein Band oder mehrere Bänder ausgebildet werden, wobei sich ein jeweiliges Band vom Verschlusskörper weg erstreckt, ggf. z.B. in einer Schlaufe zum Verschlusskörper zurückgeführt wird und hierdurch eine korbartige Struktur ausbildet, die sich von innen an die Herzwand, insbesondere das Atriums anlegen kann.

Wenngleich eine solche Befestigungsart auch in Verbindung mit der hier nachfolgend beschriebenen Erfindung bevorzugt ist, kann die vorliegende Erfindung auch bei anderen Ausführungsarten des Verankerungselementes eingesetzt werden, insbesondere auch bei solchen, die eine Penetration der Herzwand vorsehen.

Problematisch ist es bei den implantierbaren Vorrichtungen dieser Art, dass der Verschlusskörper der Vorrichtung den verbleibenden Restspaltbereich zwischen den sich schließenden Segelklappen einer Herzklappe während der Herztätigkeit möglichst passend ausfüllen muss, um Fehlströmungen des Blutes im Herzen zu verhindern.

US 2009048668 **offenbart ein System und ein Verfahren zur Implantation eines Herzimplantats.**

US 2007293943 **offenbart eine medizinische Vorrichtung zur Verwendung bei der Behandlung eines Ventils.**

US2015073547 **offenbart einen mitralen Ballonabstandshalter.**

US2013325110 **offenbart Systeme und Verfahren zum Platzieren eines Koaptierenden Elements zwischen Klappenblättchen.**

US2011034764 **offenbart Anzeigeinstrument und Herstellungsverfahren dafür.**

US2005177131 **offenbart Kathetersegment mit abwechselnden Schnitten.**

US6053922 **offenbart eine flexible Welle.**

EP2762190 **offenbart ein geschlitztes Rohr und Führungsdraht mit demselben.**

US2006241745 **offenbart eine Vorrichtung zur Kontrolle des Blutflusses.**

Im Stand der Technik ist es bekannt, beispielsweise aus der Publikation US 2006 / 00241745 A1 , dass an einem solchen Verschlusskörper bewegliche taschenförmige Elemente angeordnet sind, die sich durch die Blutströmung während der Herzphasen periodisch entfalten und wieder zusammenfalten können. Hiermit soll unter anderem bezweckt werden, dass während eines Schließens der Segelklappen der Herzklappe diese Taschen sich entfalten, hierdurch quasi den Segelklappen entgegen kommen und sich die Segelklappen sodann dicht an die Außenbereiche der entfalteten Taschen anlegen. Während des Öffnens der Segelklappen hingegen sollen die Taschen zusammen fallen und einen maximalen Blutstrom ermöglichen. Als problematisch erweist es sich hierbei, dass eine Entfaltung und das Zusammenfalten durch den Blutstrom selbst bewirkt werden soll, der Blutstrom jedoch z.B. bei unterschiedlichen Belastungen des Körpers unterschiedlich ist, demnach auch die Entfaltung nicht immer gleich ist und hierdurch entweder ein Restspalt verbleiben kann oder durch die Taschen ein Druck auf die Kanten der Segelklappen ausgeübt wird.

Darüber hinaus ist es ein Problem, dass durch die Taschen Bereiche am Verschlusskörper gebildet werden, in welche Blut hineinströmt, dort jedoch stagnieren kann, so dass damit einhergehende Risiken, z.B. Tromboserisiken nicht ausgeschlossen werden können.

Vor diesem Hintergrund ist es eine Aufgabe, eine implantierbare Vorrichtung der eingangs genannten Art insoweit zu verbessern, dass der Verschlusskörper insbesondere hinsichtlich seines Querschnittes bevorzugt der Querschnittsgröße senkrecht zur Erstreckungsrichtung zwischen Ventrikel und Atrium angepasst werden kann, um den gewünschten Verschluss des verbleibenden Restspaltbereiches zwischen den sich schließenden Segelklappen genügend auszufüllen, ohne jedoch Stagnationsbereiche auszubilden.

Diese Aufgabe wird erfindungsgemäß dadurch gelöst, dass der Verschlusskörper der implantierbaren Vorrichtung wenigstens einen Hohlraum umfasst, der nach Implantation des Verschlusskörpers mit einem Fluid befüllbar ist, bevorzugt der Verschlußkörper nicht selbsttätig entleerbar ist und in der bevorzugten Ausführung der Verschlusskörper durch die Füllung während der Herzphasen volumenkonstant expandiert bleibt.

Die Erfindung ist definiert durch Anspruch 1. Bevorzugte Ausführungsformen sind durch die abhängigen Ansprüche definiert.

Der wesentliche Kerngedanke der Erfindung ist darin zu sehen, dass durch das Auffüllen des wenigstens einen Hohlkörpers und dessen Expansion der Querschnitt des Verschlusskörpers, insbesondere betrachtet senkrecht zur Erstreckungsrichtung zwischen Ventrikel und Atrium, in der Größe je nach aufgenommenen Volumen im Hohlraum angepasst werden kann, so dass durch die Befüllung nach der Implantation die individuelle Anpassung des Verschlusskörpers an die Gegebenheiten beim Patienten vorgenommen werden kann.

Dabei wird durch die Tatsache, dass der gefüllte Hohlraum des Verschlusskörpers in der bevorzugten Ausführung nicht selbsttätig entleerbar ist sichergestellt, dass der Verschlusskörper nach einer Befüllung sein Volumen beibehält, insbesondere ein Fluid, das zur Füllung des Verschlusskörpers verwendet wird, nicht in den Körper des Patienten gelangen kann.

So werden Stagnationseffekte am oder im Verschlusskörper der erfindungsgemäßen implantierbaren Vorrichtung vermieden, sowie eventuelle damit einhergehende Thrombenrisiken.

Durch die erzielte Volumenkonstanz während der Herzphasen wird weiterhin bewirkt, dass selbst bei unterschiedlichen Belastungen des Patienten, z.B. also unabhängig davon, ob sich der Patient in einer Ruhephase befindet oder gerade Sport treibt in jedem Fall der Restspaltbereich zwischen den Segelklappen der Herzklappe immer optimal durch den Verschlusskörper ausgefüllt bleibt.

Die Vorrichtung kann dabei vorsehen, dass trotz der Volumenkonstanz die Möglichkeit besteht, dass der Hohlraum des Verschlusskörpers verschiedene Volumina einnehmen kann, z.B. dadurch dass ein bereits gefüllter Verschlusskörper, der ein bestimmtes erstes Volumen angenommen hat, nachträglich über das Volumen der Erstbefüllung hinaus nachbefüllt wird oder aber auch, dass Fluid nachträglich aus dem Verschlusskörper wieder entfernt wird, um das eingenommene Volumen zu verringern.

Hierdurch besteht die Möglichkeit, auf verschiedene Entwicklungen des Patienten einzugehen. Verschlechtert sich beispielsweise die Herzklappeninsuffizienz derart, dass sich der Spaltbereich zwischen den Segelklappen des Patienten mit fortschreitender Zeit vergrößert, so kann es die Vorrichtung vorsehen, dass der implantierte Verschlusskörper der erfindungsgemäßen Vorrichtung nachbefüllt wird, um hiernach wieder sicherzustellen, dass der Spaltbereich wieder vollständig geschlossen wird während der Herzphasen.

Somit bedarf es bei einer solchen Verschlechterung der Herzklappeninsuffizienz nicht einer Neuimplantation einer ggf. anderen Vorrichtung, sondern unter Umständen lediglich eines minimalinvasiven Eingriffs, um eine Nachbefüllung des Hohlraumes im Verschlußkörper zu erzielen. In gleicher weise kann durch eine Reduktion des Volumens durch Entnahme von zuvor eingefülltem Fluid auf eine Verbesserung der Herzklappeninsuffizienz reagiert werden.

Die Erfindung kann im Wesentlichen vorsehen, dass der Verschlusskörper der implantierbaren Vorrichtung eine Länge aufweist, so dass ein Ende des Verschlusskörpers im Ventrikel auf der einen Seite der Herzklappe und das andere Ende des Verschlusskörpers nach einer Implantation im Atrium auf der anderen Seite der Herzklappe angeordnet ist.

Um zu gewährleisten, dass durch eine Befüllung des wenigstens einen Hohlraumes des Verschlusskörpers eine genügende Abdichtung des Restspaltbereiches bei geschlossenen Herzklappen erzielt wird sieht die Erfindung es vor, dass der füllbare Hohlraum bezogen auf die Längserstreckungsrichtung des Verschlusskörpers so an diesem positioniert ist, dass die Segelklappen diesen beim Schließen kontaktieren. Um die Positionierung des Verschlusskörpers zu erleichtern kann es dabei vorgesehen sein, das der wenigstens eine Hohlraum ebenso in der Längserstreckungsrichtung des Verschlusskörpers somit also in der Verbindungsrichtung zwischen Ventrikel und Atrium erstreckt ist und somit selbst bei leicht variierten Positionierungen des Verschlusskörpers immer gewährleistet ist, dass die sich schließenden Segelklappen gegen den gefüllten Hohlraum, insbesondere dessen äußere Wandbereiche anlegen. Hierfür kann es bevorzugt vorgesehen sein, dass der Hohlraum in seiner Längserstreckung eine Länge aufweist, die wenigstens 50% der Länge des Verschlusskörpers zwischen dem Ventrikel- und atriumseitigen Enden entspricht. Besonders bevorzugt kann es hier vorgesehen sein, dass der Hohlraum beidseitig der Mitte des Verschlusskörpers bezogen auf die Längserstreckungsrichtung angeordnet ist.

In einer vorteilhaften Ausgestaltung der Vorrichtung kann es weiterhin vorgesehen sein, dass der Verschlusskörper einen einzigen Hohlraum aufweist, der einen in der Längserstreckungsrichtung des Verschlusskörpers vorgesehen Grundkörper des Verschlusskörpers umgibt. Ein solcher Grundkörper, kann z.B. rohrförmig ausgestaltet sein oder aber auch in seinem Querschnitt an eine Grundform eines Spaltbereichs zwischen den Segelklappen angepasst sein, z.B. eine Sichelform im Querschnitt aufweisen. Ein solcher Grundkörper kann beispielsweise innerhalb des Hohlraumes mittig zu diesem angeordnet sein. Die Hohlraumwände, die den Hohlraum nach außen umgrenzen, können den Grundkörper umgeben. Bezogen auf die jeweiligen Mittenachsen von Grundkörper und Hohlraum können diese koaxial angeordnet sein.

Die Erfindung kann jedoch auch vorsehen, mehrere Hohlräume um einen in der Erstreckungsrichtung zwischen Ventrikel und Atrium liegenden Grundkörper des Verschlusskörpers herum anzuordnen, insbesondere wobei in einer Ausführungsform es vorgesehen sein kann, dass jeder von mehreren Hohlräumen individuell befüllt werden kann, somit also die Hohlräume auch unterschiedlich befüllt werden können, was eine gezieltere Anpassung des von allen Hohlräumen insgesamt ausgefüllten Querschnittes des Restspaltbereiches zwischen den Segelklappen ermöglicht. An dem Grundkörper kann das Verankerungselement angeordnet sein, insbesondere an eines seiner beiden Enden anschließen.

Ein Grundkörper kann in einfacher Ausgestaltung durch ein starres Rohr ausgebildet sein, welches von dem wenigstens einen Hohlraum umgeben ist. Eine bevorzugte Ausführung kann auch vorsehen, dass der Grundkörper als ein aus seiner linearen Erstreckung herausbiegbares Element ausgebildet ist, insbesondere das durch innere Federkräfte in die lineare Erstreckung zurück relaxierbar ist. Ein solcher Grundkörper ist bevorzugt demnach selbst rückstellend flexibel. Bei einer Ausbildung als insbesondere metallischer rohrförmiger Körper kann der Grundkörper zumindest abschnittsweise zu einer Feder gewickelt sein, z.B. aus einem Draht mit rundem oder auch einem Band mit eckigem Querschnitt.

Ein biegbarer Grundkörper kann auch aus einem ursprünglich starren Rohr hergestellt sein, durch das Einbringen einer Vielzahl von Einschnitten in die Mantelfläche des rohrförmigen Grundkörpers.

Eine solche Vielzahl der Einschnitte kann in einer möglichen Ausführung wenigstens eine gegebene Anzahl von wenigstens zwei Einschnitten umfassen, die mit ihrer geometrischen Anordnung zueinander wenigstens ein Einschnitt- Muster ausbilden, wobei in der Mantelfäche des Grundkörpers mehrere Einschnitt- Muster, insbesondere gleiche oder verschiedene Einschnittmuster angeordnet sind. Eine gebildetes Einschnitt-Muster kann dabei in axialer Richtung und/oder Umfangsrichtung des Grundkörpers mehrfach, wenigstens zweimal wiederholt angeordnet sein.

Hier kann es vorgesehen sein, dass durch eine erste Anzahl von wenigstens zwei Einschnitten ein erstes Einschnitt-Muster und durch eine zweite Anzahl von wenigstens zwei Einschnitten ein zum ersten Einschnitt-Muster verschiedenes zweites Einschnitt-Muster gebildet wird. Jedes dieser Einschnitt-Muster kann auf der Mantelfläche mehrfach angeordnet sein. Die Anzahl verschiedener Muster kann auch größer als zwei sein, wobei jedes Muster wenigstens zwei Einschnitte umfasst.

Bei verschiedenen Einschnitt-Mustern kann jedes der verschiedenen Einschnitt-Muster auch nur einmal in der Mantelfläche des Grundkörpers vorkommen.

Nebeneinander liegende gleiche oder auch verschiedene Einschnitt-Muster können ohne Abstand aneinander angrenzen, aber auch einen axialen oder in Umfangsrichtung gegebenen Abstand, insbesondere äquidistanten Abstand aufweisen. Bei der letzteren beabstandeten Ausführung kann sich somit auch eine optisch erkennbare Abgrenzung der Einschnitt-Muster sogar bei mehrfach wiederholten gleichen Einschnitt-Mustern zueinander ergeben. Abstände zwischen Einschnitten und / oder Einschnitt-Mustern können auch variierend ausgebildet sein, z.B. in axialer Richtung und/oder Umfangsrichtung zunehmend und/oder wieder abnehmend oder umgekehrt ausgebildet sein.

Die Schnitterstreckungen bzw. Schnittrichtungen der Einschnitte, insbesondere innerhalb eines gebildeten Musters, können immer gleich sein oder variabel sein, insbesondere sich von Einschnitt zu Einschnitt in Richtung der axialen Erstreckung oder in Richtung des Umfangs ändern.

Der Begriff Einschnitt impliziert nicht, dass die Schnittrichtung bzw. Erstreckung eines Einschnittes immer geradlinig ist, wenngleich dies eine bevorzugte Ausführung ist. Einschnitte können gemäß der Erfindung auch hinsichtlich der Schnittrichtung gebogen ausgeführt sein. Insbesondere durch das Verfahren des Laserschneidens können Freiformen von Schnittrichtungen in der Mantelfläche des Grundkörpers realisiert sein, somit also z.B. gerade oder ein- oder mehrfach gebogene, z.B. auch wellenförmige oder teilkreisförmige Einschnitte.

Gerade und gebogene Einschnitte können miteinander kombiniert sein und z.B. im oben genannten Sinn auch ein Muster bilden. Insbesondere bei gebogenen Einschnitten können die Einschnitte ineinander verschränkt sein. Darunter wird bevorzugt verstanden, dass ein Ende wenigstens eines gebogenen Einschnittes oder auch wenigstens eines geraden Einschnittes zwischen zwei Enden eines gebogenen Einschnittes angeordnet ist.

Einschnitte, insbesondere geradlinig erstreckte Einschnitte, können, unabhängig davon, ob sie eine Muster-Anordnung aufweisen oder nicht, in bevorzugter Ausführung zumindest zum Teil (also eine Teilanzahl aller Einschnitte), ggfs. vollständig (also alle Einschnitte) senkrecht zur Längserstreckung des Grundkörpers liegen und sind bevorzugt in Längserstreckung zumindest bereichsweise oder insgesamt äquidistant beabstandet. Es kann also auch Bereiche mit unterschiedlichen Abständen, jedoch im jeweiligen Bereich äquidistanten Abständen zwischen den Einschnitten geben. In Längsrichtung aufeinander folgende Einschnitte können auch abwechselnd in unterschiedlichen Richtungen erstellt sein, worunter bevorzugt verstanden wird, dass Schnitte bezogen auf einen in Umfangsrichtung betrachteten Winkel bei verschiedenen Winkelpositionen erstellt sind. Die Winkelpositionen können äquidistant sein, z.B. liegen Schnitte bevorzugt bei 0 Grad, 90 Grad, 180 Grad und 270 Grad.

An einer jeweiligen Längsrichtungsposition können sich auch jeweils zwei Schnitte gegenüberliegen, die jeweils vor der Mitte des Rohres enden, z.B. ein Paar von gegenüberliegenden Schnitten bei 0 und 180 Grad sowie ein Paar bei 90 und 270 Grad. Die Paare sind sodann in Längsrichtung beabstandet, bevorzugt äquidistant. Solche Paare können z.B. auch als Einschnitt-Muster im oben genannten Sinn verstanden werden.

Eine sich selbst rückstellende Biegbarkeit eines rohrförmigen Grundkörpers kann auch durch einen oder mehrere helikale oder mäanderförmige Schnitte in der Mantelfläche des Grundkörpers realisiert sein. Bei mehreren Schnitten können diese ineinander verschränkt sein. Ein solcher Mäander-Schnitt kann abwechselnd in Längsrichtung und quer, bevorzugt senkrecht dazu verlaufende Schnitt- Abschnitte aufweisen. Unabhängig davon, welche Anzahl von Hohlräumen bei der erfindungsgemäßen Ausgestaltung der implantierbaren Vorrichtung am Verschlusskörper vorgesehen ist kann die Erfindung vorsehen, dass der wenigstens eine Hohlraum zumindest bereichsweise und insbesondere zumindest die äußeren Wandbereiche durch ein flexibles Wandmaterial ausgebildet ist.

So kann der Hohlraum z.B. mittels eines flüssigen Fluides befüllt werden, ohne Gasblasen in diesem auszubilden, wofür es beispielsweise vorgesehen sein kann, dass der Hohlraum vor einer Erstbefüllung vollständig kollabiert ist, ohne einen gasförmigen Inhalt aufzuweisen. Z.B. kann es dafür vorgesehen sein, den Hohlraum mit einem flüssigen Fluid vor der Implantation vorzubefüllen, und sodann dieses Fluid wieder derart aus dem Hohlraum zu verdrängen, dass sämtliche gasförmigen Einschlüsse entfernt werden, so dass hiernach die implantierte Vorrichtung im Herzen eines Patienten sodann blasenfrei befüllt werden kann.

Die Erfindung kann hier auch vorsehen, dass das flexible Wandmaterial dehnbar ist, um eine Befüllung mit unterschiedlichen Volumina trotz gestraffter Wandbereiche zu erzielen. Dafür kann ein Wandbereich z.B. aus einem biokompatiblen Silikon ausgebildet sein.

Insbesondere um eine Begrenzung des maximal einfüllbaren Volumens zu erzielen, kann hier die Erfindung vorsehen, dass der wenigstens eine Hohlraum, insbesondere an oder in dessen Wandbereich, von einer flexiblen jedoch nicht dehnbaren Struktur, beispielsweise einer Geflechtstruktur überdeckt ist. So kann bei einer solchen Ausführung der Hohlraum nicht über ein maximales Volumen hinaus befüllt werden, da die nicht dehnbare, den Hohlraum bevorzugt außen umgebende Struktur einer Weiterbefüllung entgegen wirkt.

Eine solche nicht dehnbare Struktur kann auf einem Wandmaterial des Hohlraumes z.B. außen angebracht sein, es kann jedoch auch vorgesehen sein, eine solche nicht dehnbare Struktur in einen mehrschichtigen Wandbereichaufbau eines Hohlraumes einzubetten.

Die Erfindung sieht in bevorzugter Ausführung vor, dass der Verschlusskörper wenigstens einen Verschluß aufweist, durch welchen der wenigstens eine Hohlraum aktiv oder auch passiv füllbar ist.

In einer Ausführung kann es hier vorgesehen sein, dass ein solcher Verschluß z.B. an einem, bei mehreren Hohlräumen ggf. auch an jedem Hohlraum angeordnet ist, um die Befüllung durch Öffnen des Verschlusses zu ermöglichen.

In allen Ausführungen kann der Verschluß beispielsweise durch eine von außen ansetzbare Handhabe, insbesondere die an einem Katheter angeordnet ist, geöffnet und geschlossen werden, so dass ein Chirurg dieses jeweils wahlweise bewirken kann. Der Verschluß kann dafür als Ventil ausgebildet sein, das wahlweise geöffnet oder geschlossen werden kann.

Die Erfindung kann jedoch auch vorsehen, dass der wenigstens eine vorgesehene Verschluß als ein Einwegventil ausgebildet ist oder ein solches umfasst, somit also nur Fluid durch das Ventil in den Hohlraum einströmen jedoch nicht zurück aus dem Hohlraum herausströmen kann.

Ein solches Einwegventil muss nicht von außen zwischen einer geöffneten oder geschlossenen Stellung umgesteuert werden, sondern lässt es insbesondere immer zu, eine Befüllung vorzunehmen, sofern der von außen an dem Ventil anstehende Fluiddruck größer ist, als derjenige Druck der zum Öffnen des Ventils benötigt wird.

Ein Verschluss, der wie vorbenannt als Ventil ausgeführt sein kann, kann beispielsweise an dem zum Ventrikel gerichteten Ende des Verschlusskörpers angeordnet sein, z.B. in einer Wandung eines Hohlraumes oder gemäß einer anderen Ausführungsform auch an einem Ende des von dem wenigstens einen Hohlraum umgebenden hohlen Grundkörpers, wobei es dann auch vorgesehen sein kann, dass der Grundkörper ein Durchgangselement ausbildet, durch welches hindurch die Befüllung des wenigstens einen Hohlraumes erfolgen kann. Der Grundkörper kann sodann einen Kanal bilden, der den Verschluß mit dem Inneren des wenigstens einen Hohlraumes verbindet.

Der Verschluss kann insbesondere auch so ausgebildet sein, dass nicht nur eine Befüllung des wenigstens einen Hohlraumes möglich ist, sondern auch eine Entleerung, so dass z.B. eine nachträgliche Entfernung der erfindungsgemäßen implantierbaren Vorrichtung problemlos möglich ist nach vorheriger Entfernung des Fluids.

Die Vorrichtung kann hier allgemein vorsehen, dass für die Durchführung einer aktiven Befüllung mit einem bevorzugt flüssigen Fluid an oder durch den Verschluss eine Füllhilfe steckbar ist. Eine solche Füllhilfe kann z.B. durch einen Katheter gebildet werden, durch welchen hindurch ein bevorzugt flüssiges Fluid in den Verschlusskörper, also in den wenigstens einen Hohlraum einfüllbar ist, so dass dieser durch die Befüllung expandiert.

Die Erfindung sieht jedoch vor, dass eine passive Befüllung durch den Verschluss hindurch erfolgt, z.B. während der Herztätigkeit durch die bei der Herztätigkeit erzeugten hydrodynamischen oder hydrostatischen Druckverhältnisse.

So ist durch Ausbildung des Verschlusses als Einwegventil dieses so konfiguriert, dass durch den anstehenden hydrostatischen oder hydrodynamischen Druck der Verschluss mit Blut durchströmbar ist, somit also nach einer Implantation der erfindungsgemäßen Vorrichtung eine automatische Befüllung des wenigstens einen Hohlraumes des Verschlusskörpers erfolgt.

Besonders bei dieser letztgenannten Ausführungsform, aber auch bei einer aktiven Befüllung mit Blut kann es auch vorgesehen sein, dass in dem wenigstens einen Hohlraum insbesondere bereits vor einer Implantation ein die Blutgerinnung bewirkender Stoff angeordnet ist. So wird hierdurch erzielt, dass unabhängig davon, ob eine aktive oder passive Befüllung eines Hohlraumes mit dem Blut des Patienten erfolgt, nach der Befüllung eine Blutgerinnung einsetzt und sich das Blut verfestigt.

Die Vorrichtung kann in anderer, nicht beanspruchten Ausführung auch vorsehen, dass der wenigstens eine Hohlraum mit einem expandierbaren, wie beispielsweise quellbarem Fluid füllbar ist. Ein solches Fluid kann beispielsweise auch bereits vor einer Implantation in dem Hohlraum angeordnet sein, jedoch vor der Implantation noch nicht expandiert z.B. gequollen sein, um die Implantation zu erleichtern.

Hier kann die Vorrichtung vorsehen, dass die Expansion des expandierbaren Fluides gezielt auslösbar ist, um sicherzustellen, dass eine Expansion, beispielsweise ein Quellen des Fluides, erst erfolgt, nachdem die erfindungsgemäße Vorrichtung korrekt platziert und implantiert ist. Beispielsweise kann dies dadurch erreicht werden, dass auf das in dem Hohlraum angeordnete Fluid ein Initiator einwirkt, der durch einen offenbaren Verschluss des wenigstens einen Hohlraumes in diesen einfüllbar ist.

Es besteht somit beispielsweise lediglich die Notwendigkeit, dass nach der Implantation einer erfindungsgemäßen Vorrichtung eine geringe Menge an Initiator in den Hohlraum eingefüllt werden muss, um die Expansion des Fluides zu starten.

Die Vorrichtung kann jedoch auch vorsehen, dass eine Initiierung der Expansion insbesondere eine Initiierung des Quellens des im Hohlraum bereits anfänglich vorhandenen oder nach der Implantation eingefüllten Fluides dadurch erfolgt, dass die Wandung des Hohlraums für den Initiator permeabel ausgebildet ist.

Beispielsweise kann es hier vorgesehen sein, dass als Initiator ein Blutbestandteil genutzt wird, der durch die Wandung des Hohlraumes hindurch treten kann.

Beispielsweise kann hierfür der Wasseranteil des Blutes genutzt werden, so dass eine Expansion des quellbaren Fluides beispielsweise dadurch bewirkt werden kann, dass Feuchtigkeit aus dem Blut durch die Wandung in den Hohlraum übertritt und dort das expandierbare Fluid zur Expansion auslöst.

Ausführungsformen der Erfindung werden anhand der nachfolgenden Figuren näher beschrieben.

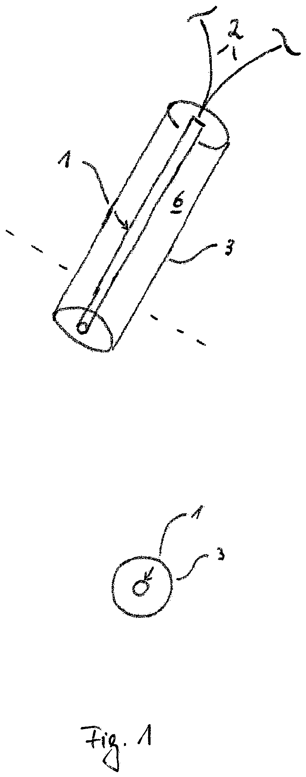

Figur 1 zeigt ausschnittsweise eine implantierbare Vorrichtung gemäß der Erfindung, in welcher der Verschlußkörper einen Grundkörper 1 aufweist, der hier im vorliegenden Fall rohrförmig ausgebildet ist, wobei am hier oberen Ende an den Verschlusskörper ein Ankerelement 2 angeordnet ist, das hier durch Bänder realisiert ist, die sich nach Implantation von innen an die Herzwandung des Atrium anlegen können und so den Verschlusskörper insgesamt ohne Penetration von Herzgewebe in der Position fixieren. Das Ankerelement 2 ist hier nur ansatzweise gezeigt, da es kein wesentlicher Bestandteil der Erfindung ist. Der hier gezeigte Grundkörper kann gemäß vorher beschriebenen Ausführungen Einschnitte zur Erzielung einer reversiblen Biegbarkeit umfassen, diese Einschnitte sind hier jedoch nicht visalisiert.

Die erfindungsgemäße Ausgestaltung ist hier derart, dass um den Grundkörper 1 , der als Rohr ausgebildet ist, eine flexible Wandung 3 angeordnet ist, die sowohl im unteren als auch oberen Bereich gedichtet an den Grundkörper 1 anschließt und einen Hohlraum 6 umgrenzt, der durch innere Befüllung mit einem bevorzugt flüssigen Fluid im Volumen expandierbar ist. Hierfür ist die Wandung 3 des Hohlraumes 6 flexibel ggfs. sogar elastisch ausgestaltet.

Die Darstellung der Figur 1 unten, die den Querschnitt des Verschlußkörpers am Ort der gestrichelten Linie zeigt, verdeutlicht, dass der Querschnitt im gefüllten Zustand rund sein kann. Es ist aber auch möglich den Querschnitt elliptisch oder sichelförmig auszubilden. Die axialen Enden des Hohlraumes 6 können durch Wandbereiche senkrecht oder quer zur Längserstreckung ausgebildet sein.

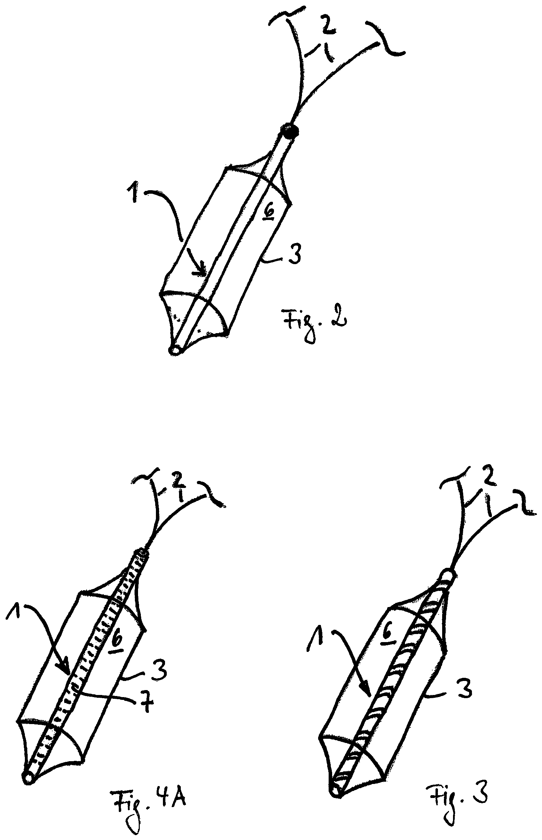

Die Figuren 2 bis 5 zeigen eine Vorrichtung, bei welcher die äußere Hohlraumwandung 3 an den jeweiligen axialen Enden des Hohlraumes verjüngend auf den Grundkörper 1 zuläuft und an diesem befestigt ist.

Figur 2 verdeutlich hierbei eine Ausführung, bei welcher der Grundkörper 1 als starres Rohr ausgebildet ist.

Figur 3 zeigt eine Ausführung, bei welcher der Grundkörper 1 zumindest bereichsweise aus einem Band zu einer Spirale bzw. Feder gewickelt ist. Statt eines gewickelten Bandes kann der Grundkörper in seiner Mantelfläche auch einen einzigen Einschnitt aufweisen, der sich helikal um den Grundkörper herumwindet.

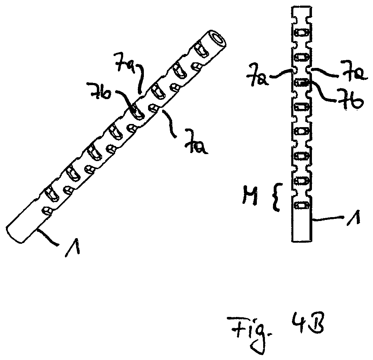

Figur 4A zeigt eine Ausführung, bei welcher in ein ursprünglich starres Rohr mehrere in Längsrichtung beabstandete Paare von sich gegenüberliegenden Einschnitten 7 angeordnete sind. Dies ist im Detail besser In der Figur 4B nur am Beispiel des Grundkörpers 1 dargestellt ohne die umgebenden konstruktiven Elemente zu zeigen. Die Einschnitt 7a bilden hierbei ein erstes Paar von gegenüberliegenden Einschnitten und die Einschnitte 7b ein zweites Paar.

In Längsrichtung aufeinanderfolgende Paare von sich gegenüberliegenden Einschnitten 7 sind jeweils um 90 Grad versetzt zueinander. Die sich gegenüberliegenden Paare von Einschnitten verlaufen jeweils in radialer Richtung von der Mantelfläche bis vor die Rohrmitte des Grundkörpers 1 , so dass sich die Schnitte nicht treffen und ein Mantelflächenbereich zwischen diesen verbleibt. Diese verbleibenden Mantelflächenbereiche bilden jeweils eine Art

Festkörpergelenk. Durch die Vielzahl der Schnitte wird der Grundkörper 1 biegbar und relaxiert in die lineare Grundstellung bevorzugt selbsttätig.

Die Anordnung aus den beiden Paaren der Einschnitte 7a und 7b kann auch als ein Muster M einer Anzahl von 4 Einschnitten verstanden werden. Dieses Muster M wiederholt sich entlang der axialen Erstreckung mehrfach, hier mit äquidistantem Abstand.

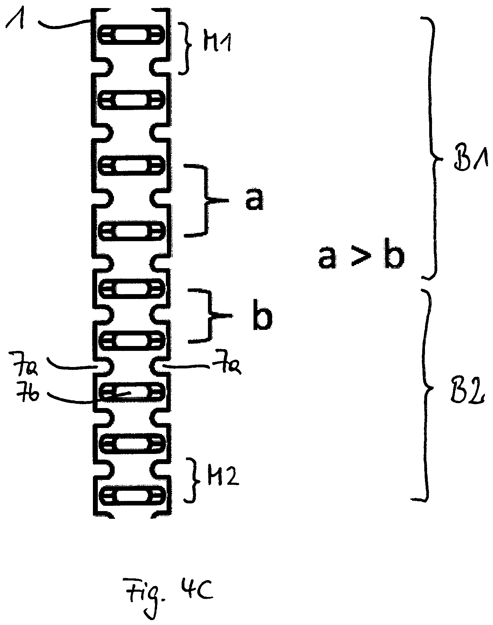

Figur 4C visualisiert eine Abwandlung, bei der ebenso Paare von gegenüberliegenden Einschnitte 7a bzw. 7b gebildet sind, wobei die Paare wiederum zueinander um 90 Grad in Umfangsrichtung versetzt und axial beabstandet sind. Die Schnittführung ist wieder so, dass sich die gegenüberliegenden Einschnitte nicht treffen, insbesondere also vor der Rohrmitte enden. Hier ist erkennbar, dass durch solche Paare ein erstes Muster M1 und ein zweites Muster M2 gebildet sein kann. Jedes der Muster ist wiederholt in axialer Richtung hintereinander angeordnet. Das Muster M1 wiederholt sich äquidistant mit dem Abstand a und das Muster M2 wiederholt sich äquidistant mit dem Abstand b. In axialer Richtung können Bereiche B1 des ersten Abstandes a und Bereiche B2 des zweiten Abstandes b wenigstens einmal, bevorzugt mehrfach aufeinander folgend angeordnet sein.

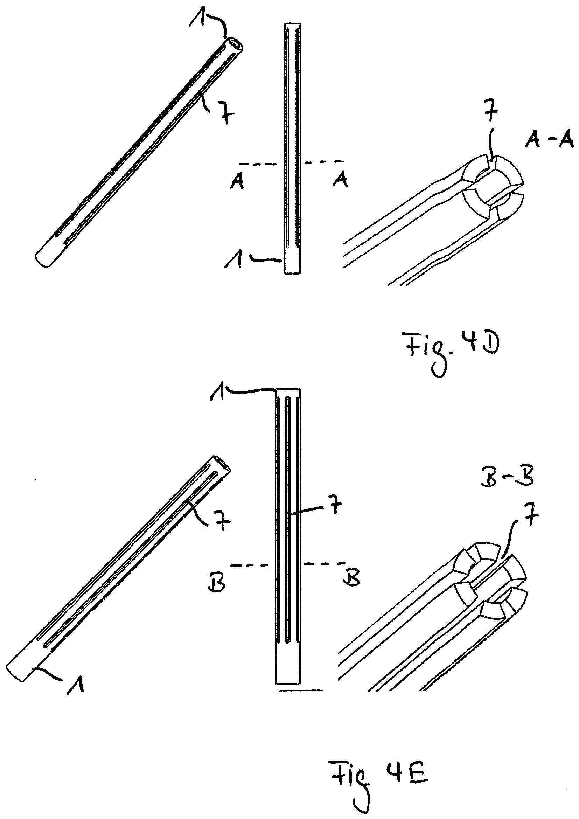

Die Figuren 4D und 4E zeigen jeweils eine Ausführung mit Einschnitten 7, die jeweils in axialer Längsrichtung verlaufen. In Umfangsrichtung sind bei Figur 4D vier Einschnitte und bei Figur 4E sechs Einschnitte 7 vorgesehen. Die Anzahl der Einschnitte 7 kann grundsätzlich beliebig sein, bevorzugt jedoch wenigstens vier. Die Einschnitte 7 haben unabhängig von der möglichen Anzahl bevorzugt zueinander alle in Umfangsrichtung den gleichen Winkelabstand. Alle Einschnitte enden jeweils vor den axialen Enden des Grundkörpers 1. Die rechtseitigen Ansichten beider Figuren zeigen jeweils Aufsichten auf die markierten Schnittebenen A-A bzw. B-B.

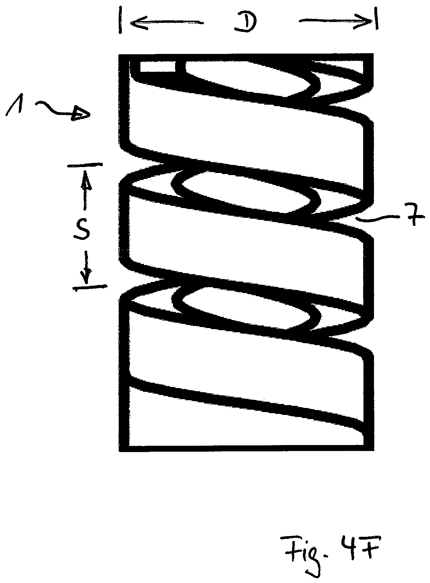

Figur 4F zeigt eine Alternative, bei welcher der Grundkörper 1 , der hier nur ausschnittweise gezeigt ist, einen einzigen Einschnitt 7 in der Mantelfläche aufweist, der sich helikal, also schraubenförmig um die Längsachse windet. Auch hier endet der Einschnitt vor den jeweiligen axialen enden des Grundkörpers. Die Steigung S ist hier (pro Umdrehung) kleiner als der Außendurchmesser D des Grundkörpers 1.

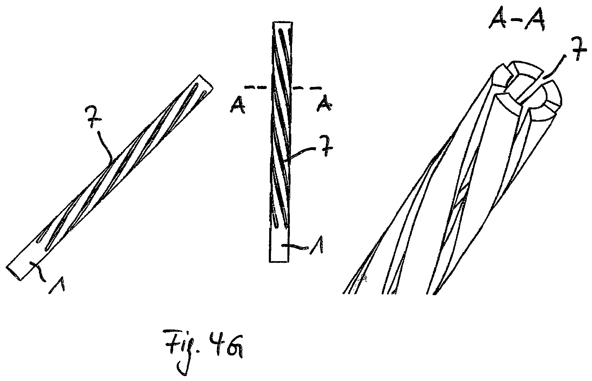

Figur 4G zeigt eine Ausführung, bei der in der Mantelfläche des Grundkörpers mehrere (wenigstens zwei) Einschnitte 7 mit helikalem Verlauf um die Längsachse angeordnet sind. Alle Einschnitte 7 enden wiederum vor den beiden axialen Enden des Grundkörpers 1. In dieser Figur sind beispielsweise fünf Einschnitte 7 vorgesehen. Sie haben zueinander denselben Winkelabstand in der Umfangsrichtung. Die Anzhal ist beliebig, jedoch mindestens zwei.

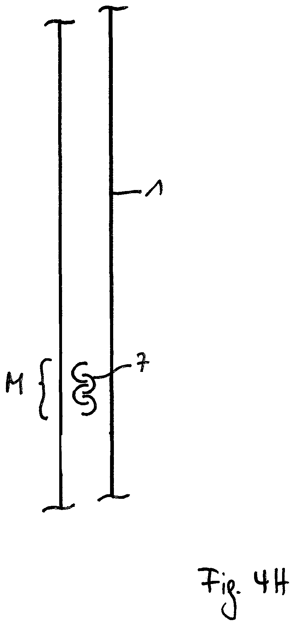

Figur 4H zeigt eine Variante, bei der die Einschnitte 7 jeweils gebogen ausgeführt sind. Das Ende eine Einschnittes 7 liegt hier zwischen den Enden eines anderen Einschnittes. Solche Einschnitte, hier z.B. die gezeigten 4 Einschnitte 7 können wierderum ein Muster M bilden, das sowohl in axialer Richtung als auch in Umfangsrichtung wiederholt angeordnet ist. Die Wiederholungen sind hier nicht visualisiert. Bei allen Ausführungen wird unter einem Einschnitt ein solcher Schnitt verstanden, der komplett durch die Wandungsdicke der Rohrwandung des hohlen rohrförmigen Grundkörpers 1 hindurchreicht.

Figur 41 zeigt eine weitere Variante, bei welcher ein mäanderförmiger Schnitt 7 in der Mantelfläche des Grundkörpers 1 realisiert ist. Von solchen mäanderförmigen Einschnitten 7 können mehrere vorgesehen sein. Diese mehreren Einschnitte können unter verschiedenen Winkeln bezogen auf die Umfangsrichtung angeordnet sein und / oder axial hintereinander liegen und/oder weiterhin auch ineinander verschränkt sein.

Ein mäanderförmiger Einschnitt 7 kann auch verstanden werden als eine Hintereinanderreihung mehrerer Einzelschnitte, bevorzugt die abwechselnd axial und in Umfangsrichtung angeordnet sind und ineinander übergehen. Allgemein können die Einzelschnitte auch unter von 90 Grad verschiedenen Winkeln zueinander liegen und/oder nicht exakt axial und nicht exakt in Umfangsrichtung, insbesondere nur jeweilige Richtungskomponenten dieser Richtungen aufweisen.

Allgemein kann weiterhin ein Mäanderschnitt auch als ein Muster M mehrerer Einschnitte 7a, 7b, 7c... verstanden werden, das sich bevorzugt wiederholt, wie es eingangs allgemein zur Beschreibung von Mustern erwähnt ist.

Bei allen vorgenannten Ausführungen kann weiterhin der nicht visualisierte Verschluß z.B. in einem Wandbereich 3 des Hohlraumes 6 angeordnet sein oder im Grundkörper 1 z.B. an dessen im Ventrikel liegenden axialen, bevorzugt offenen Ende.

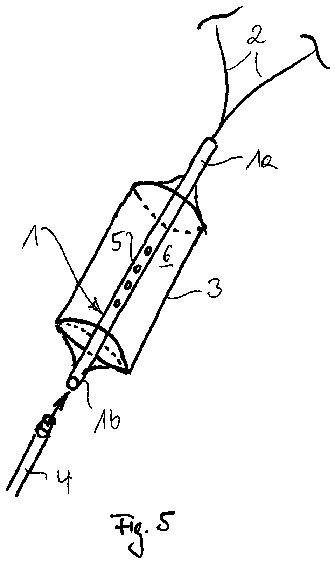

Gemäß Figur 5 kann die Erfindung vorsehen, dass das hier unten dargestellte Ende 1 b des rohrförmigen Grundkörpers 1 eine Verschlussöffnung aufweist, in die z.B. ein Einwegventil integriert ist, sodass mittels eines Katheters 4 ein Fluid, insbesondere ein flüssiges Fluid in den Grundkörper 1 injiziert werden kann.

Der Grundkörper 1 weist in der Ausführung der Figur 5 auf seiner Mantelfläche Öffnungen 5 auf, so dass das injizierte Fluid in den Hohlraum 6 eindringen kann, der durch die flexible Wandung 3 um den Grundkörper 1 herum ausgebildet ist. Der Grundkörper ist hier starr.

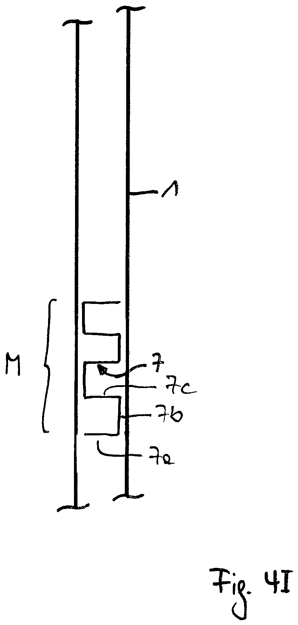

Sofern der Grundkörper flexibel ausgebildet ist können die Öffnungen 5 auch durch die Einschnitte 7 ausgebildet sein, die in den Figuren 4 gezeigt sind. Die Ausführung der Figur 5 ist mit allen Ausführungen der Figurenserie 4 kombinierbar. Es kann bei der Ausführung gemäß Figur 5 anstelle des gezeigten Grundkörpers 1 auch der spiralig gewickelte Grundkörper gemäß Figur 3, helikal geschnittene Grundkörper gemäß Figuren 4F oder 4G oder der mit gerade Einschnitten versehene Grundkörper 1 der Figuren 4A,B,C,D,E oder der mit gebogenen Einschnitten gemäß Figur 4H eingesetzt werden, um die Ausführung biegbar zu gestalten.

Bei allen Ausführungen kann je nach Menge des injizierten Fluides das Volumen des Hohlraumes 6 unterschiedlich groß ausgebildet werden und so auch insgesamt der ausgebildete Verschlusskörper hinsichtlich seiner Querschnittsgröße patientenspezifisch angepasst werden. Der einsatzfähige Verschlußkörper wird hier gebildet durch den Grundkörper 1 und den mit Fluid expandierten Hohlraum 6.

Statt der hier gezeigten Befüllung über das untere Ende 1 b des Grundkörpers 1 , die bei allen Ausführungen der Figuren 1 bis 5 realisiert sein kann, kann es ebenso vorgesehen sein, dass ein Verschluss in der Wandung 3 des Hohlraumes 6 angeordnet ist, demnach eine Befüllung mit einem bevorzugt flüssigen Fluid unmittelbar durch die Wandung 3 hindurch erfolgt. Auch hier kann die Ausbildung des Verschlusses als Einwegventil vorgesehen sein oder aber auch als ein Ventil, das zwischen der offenen und geschlossenen Stellung umsteuerbar ist.

Insbesondere durch die letztgenannte Ausführung kann es auch erzielt werden, dass das in den Hohlraum applizierte Fluid zu einem späteren Zeitpunkt wiederum entfernt werden kann, z.B. durch Ansetzen eines Katheters und Absaugung des Fluides.

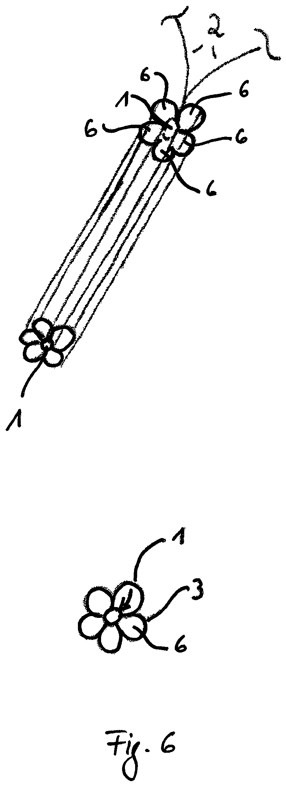

Figur 6 zeigt als weitere Ausführung die Anordnung von mehreren Hohlräumen 6, die in Umfangsrichtung um den Grundkörper 1 herum angeordnet sind. Jeder Hohlraum 6 kann ggfs. individuell oder alle gemeinsam befüllt werden. Die Hohlräume 6 können z.B. alle eine Verbindung zum hohlen Grundkörper 1 aufweisen und sodann durch diesen hindurch gemeinsam befüllt werden. Dafür kann der Grundkörper 1 den Verschluß z.B. an seinem zum Ventrikel weisenden Ende aufweisen. Bei separater Befüllung weisen die Hohlräume 6 bevorzugt jeweils einen Verschluß in der jeweiligen Wandung auf.

Wenngleich es nicht dargestellt ist können alle den Grundkörper umgebende Hohlräume zusätzlich zu ihrer jeweiligen Hohlraumwandung durch eine gemeinsame Wandung, insbesondere zumindest in Umfangsrichtung überspannt sein. In der Ausführung der Figur 6 können ebenso Grundkörper 1 gemäß der Figur 3 oder gemäß der Serie der Figuren 4 eingesetzt werden.

Als Fluid kann im Wesentlichen allgemein für die Belange der Erfindung jedes bevorzugt flüssige Fluid eingesetzt werden, das biokompatibel ist. Erfindungsgemäß ist vorgesehen das Blut des Patienten selbst zur Befüllung des wenigstens einen Hohlraumes des Verschlusskörpers zu verwenden.

## Patentansprüche

1. Implantierbare Vorrichtung zur Verbesserung oder Behebung einer Herzklappeninsuffizienz, umfassend einen Verschlusskörper, der wenigstens einen Hohlraum (6) umfasst, der nach Implantation des Verschlusskörpers mit einem Fluid befüllbar ist, und umfassend wenigstens ein an dem Verschlusskörper befestigtes Verankerungselement (2),
wobei die Vorrichtung **dadurch gekennzeichnet ist, dass** das Verankerungselement (2) am Verschlusskörper eine atraumatische Befestigung im Herzen ermöglicht, dadurch, dass sich das Verankerungselement zum Zweck der Befestigung an die Innenwand des Herzens anlegt, ohne die Herzwandung zu penetrieren,
wobei der wenigstens eine Hohlraum (6) um einen in der Längserstreckung des Verschlusskörpers liegenden Grundkörper (1) des Verschlusskörpers herum angeordnet ist,
und der wenigstens eine füllbare Hohlraum bezogen auf die Längserstreckungsrichtung des Verschlusskörpers so an diesem positioniert ist, dass die Segelklappenihn beim Schließen kontaktieren,
wobei der Grundkörper (1) als Rohr ausgebildet ist und um den Grundkörper eine flexible Wandung (3) angeordnet ist, die sowohl im unteren als auch oberen Bereich gedichtet an den Grundkörper (1) anschließt und den wenigstens einen Hohlraum (6) umgrenzt, der durch innere Befüllung mit einem bevorzugt flüssigen Fluid im Volumen expandierbar ist, wobei an dem einen oder mehreren Hohlraum ein als Einwegventil ausgebildeter Verschluss angeordnet ist, der durch den anstehenden hydrostatischen oder hydrodynamischen Druck mit Blut durchströmbar ist, so dass nach einer Implantation der Vorrichtung eine automatische Befüllung des wenigstens einen Hohlraumes erfolgt.

2. Vorrichtung nach Anspruch 1, wobei der Grundkörper (1) als ein aus seiner linearen Erstreckung herausbiegbares Rohr ausgebildet ist, in dessen Mantelfläche eine Vielzahl von Einschnitten eingebracht sind.

3. Vorrichtung nach Anspruch 2, wobei die Vielzahl der Einschnitte (7) wenigstens eine gegebene Anzahl von wenigstens zwei Einschnitten umfasst, die mit ihrer geometrischen Anordnung zueinander wenigstens ein Einschnitt-Muster ausbilden, wobei in der Mantelfläche des Grundkörpers mehrere Einschnitt-Muster, insbesondere gleiche oder verschiedene Einschnitt-Muster angeordnet sind.

4. Vorrichtung nach Anspruch 3, wobei zumindest ein Teil der Einschnitte, bevorzugt alle Einschnitte (7) senkrecht zur Längserstreckungsrichtung liegen.

5. Vorrichtung nach Anspruch 3, wobei mehrere mäanderförmige Schnitte in der Mantelfläche des Grundkörpers (1) realisiert sind.

6. Vorrichtung nach Anspruch 3, wobei der Grundkörper spiralig gewickelt ist.

7. Vorrichtung nach Anspruch 3, wobei die Einschnitte teilkreisförmig sind.

8. Vorrichtung nach Anspruch 1, wobei der wenigstens eine Hohlraum an oder in dessen Wandbereich von einer flexiblen jedoch nicht dehnbaren Struktur überdeckt ist, um eine Begrenzung des maximal einfüllbaren Volumens zu erzielen.

9. Vorrichtung nach Anspruch 1, wobei der Verschlusskörper der implantierbaren Vorrichtung eine Länge aufweist, so dass ein Ende des Verschlusskörpers im Ventrikel auf der einen Seite der Herzklappe und das andere Ende des Verschlusskörpers nach einer Implantation im Atrium auf der anderen Seite der Herzklappe angeordnet ist.

## Claims

1. An implantable device for improving or remedying valvular incompetence, comprising a sealing body which comprises at least one cavity (6), which, after implantation of the sealing body, is fillable with a fluid, and comprising at least one anchoring element (2) fastened to the sealing body,
wherein the device is **characterised in that** the anchoring element (2) on the sealing body allows atraumatic fastening in the heart, since the anchoring element for the purpose of the fastening rests against the inner wall of the heart without penetrating the wall of the heart,
wherein the at least one cavity (6) is disposed about a main body (1) of the sealing body lying in the direction of longitudinal extent of the sealing body and the at least one fillable cavity is positioned on the sealing body relative to the direction of longitudinal extent of the sealing body such that the atrioventricular valves contact the sealing body when closing,
wherein the main body (1) is formed as a tube and a flexible wall (3) is disposed around the main body and adjoins the main body (1) in a sealed manner both in the lower and upper region and delimits the at least one cavity (6), which is expandable in volume by an inner filling with a preferably liquid fluid, wherein a closure formed as a one-way valve is disposed on the one or more cavities and can be passed through by blood due to the hydrostatic or hydrodynamic pressure applied, so that, following implantation of the device, the at least one cavity is filled automatically.

2. The device according to claim 1, wherein the main body (1) is formed as a tube that can be bent out of its linear extent and in the lateral surface of which a plurality of cuts are introduced.

3. The device according to claim 2, wherein the plurality of cuts (7) comprises at least a given number of at least two cuts, which form at least one cut pattern by their geometric arrangement relative to one another, wherein a plurality of cut patterns, in particular identical or different cut patterns, are arranged in the lateral surface of the main body.

4. The device according to claim 3, wherein at least some of the cuts, preferably all of the cuts (7), lie perpendicular to the direction of longitudinal extent.

5. The device according to claim 3, wherein a plurality of meandering cuts are formed in the lateral surface of the main body (1).

6. The device according to claim 3, wherein the main body is wound in a spiralled manner.

7. The device according to claim 3, wherein the cuts are pitch-circle-shaped.

8. The device according to claim 1, wherein the at least one cavity is covered at or in its wall region by a structure which is flexible but not ductile in order to achieve the maximally fillable volume.

9. The device according to claim 1, wherein the sealing body of the implantable device has a length such that one end of the sealing body is disposed in the ventricle on one side of the heart valve and the other end of the sealing body is arranged on the other side of the heart valve following implantation in the atrium.

## Revendications

1. Dispositif implantable pour améliorer ou supprimer une insuffisance valvulaire comprenant un corps d'obturation qui comporte au moins une cavité (6) qui peut être remplie d'un fluide après implantation du corps d'obturation, et comprenant au moins un élément d'ancrage (2) fixé au corps d'obturation,
le dispositif étant **caractérisé en ce que** l'élément d'ancrage (2) sur le corps d'obturation permet une fixation atraumatique dans le coeur, **en ce que** l'élément d'ancrage s'applique contre la paroi interne du coeur à des fins de fixation, sans pénétrer dans la paroi du coeur,
la ou les cavité(s) (6) étant agencée(s) autour d'un corps de base (1) du corps d'obturation situé dans le sens de la longueur du corps d'obturation, et la ou les cavité(s) pouvant être remplie(s) étant positionnée(s) sur celui-ci, par rapport au sens de la longueur du corps d'obturation, de façon que les valvules mitrales entrent en contact avec lui lors de l'obturation,
le corps de base (1) étant réalisé sous forme de tube et une paroi souple (3) étant agencée autour du corps de base, laquelle se raccorde de manière étanche au corps de base (1) aussi bien dans la zone inférieure que dans la zone supérieure et délimite la ou les cavité(s) (6) dont le volume peut être dilaté par un remplissage interne avec un fluide, de préférence un liquide, une obturation conçue comme un clapet antiretour étant agencée sur la ou les cavité(s), laquelle peut être traversée par le sang sous l'effet de la pression hydrostatique ou hydrodynamique existante, de façon qu'après implantation du dispositif, un remplissage automatique de la ou des cavité(s) se produise.

2. Dispositif selon la revendication 1, dans lequel le corps de base (1) est réalisé sous forme d'un tube pouvant être plié sur sa longueur, dans la surface latérale duquel est pratiquée une pluralité d'entailles.

3. Dispositif selon la revendication 2, dans lequel la pluralité d'entailles (7) comprend au moins un nombre donné d'au moins deux entailles qui forment par leur disposition géométrique les unes par rapport aux autres au moins un motif d'entailles, plusieurs motifs d'entailles, en particulier des motifs d'entailles identiques ou différents, étant agencés dans la surface latérale du corps de base.

4. Dispositif selon la revendication 3, dans lequel au moins une partie des entailles, de préférence toutes les entailles (7), sont perpendiculaires au sens de la longueur.

5. Dispositif selon la revendication 3, dans lequel plusieurs découpes en forme de méandres sont réalisées dans la surface latérale du corps de base (1).

6. Dispositif selon la revendication 3, dans lequel le corps de base est enroulé en spirale.

7. Dispositif selon la revendication 3, dans lequel les entailles sont en forme d'arc de cercle.

8. Dispositif selon la revendication 1, dans lequel la ou les cavité(s) est/sont recouverte(s) sur ou dans sa/leur zone de paroi par une structure souple mais non extensible, afin d'obtenir une limitation du volume maximal pouvant être rempli.

9. Dispositif selon la revendication 1, dans lequel le corps d'obturation du dispositif implantable présente une longueur telle qu'une extrémité du corps d'obturation est située dans le ventricule d'un côté de la valvule cardiaque et que l'autre extrémité du corps d'obturation est située sur l'autre côté de la valvule cardiaque après une implantation dans l'oreillette.
